# EUROPÄISCHE PATENTSCHRIFT

(11) **EP 3 761 949 B1**
(45) Veröffentlichungstag und Bekanntmachung des Hinweises auf die Patenterteilung: **18.10.2023**
(21) Anmeldenummer: 19704600.6
(22) Anmeldetag: 08.02.2019
(51) Int. Cl.: A61K 8/49, A61Q 17/04, A61K 8/92

(54) **SONNENSCHUTZMITTEL MIT REDUZIERTER TEXTILVERFLECKUNG ENTHALTEND HYDRIERTES PFLANZENÖL UND BIS-ETHYLHEXYLOXYPHENOL METHOXYPHENYL TRIAZINE**
SUNSCREEN WITH A REDUCED TENDENCY TO STAIN TEXTILES, CONTAINING HYDROGENATED VEGETABLE OIL AND BIS-ETHYLHEXYLOXYPHENOL METHOXYPHENYL TRIAZINE
AGENT DE PROTECTION SOLAIRE AYANT UNE TENDANCE RÉDUITE À FORMER DES TACHES SUR LES TEXTILES ET CONTENANT DE L'HUILE VÉGÉTALE HYDROGÉNÉE ET DES BIS-ÉTHYLHEXYLOXYPHÉNOL MÉTHOXYPHÉNYL TRIAZINES

(30) Priorität: 08.03.2018 DE 102018203492
(43) Veröffentlichungstag der Anmeldung: 13.01.2021
(73) Patentinhaber: Beiersdorf AG, 20253 Hamburg (DE)
(72) Erfinder: SPROCK, Sarah, 22297 Hamburg (DE); LERG, Heike, 22303 Hamburg (DE)
(74) Vertreter: Beiersdorf AG
(86) Internationale Anmeldenummer: PCT/EP2019/053129
(87) Internationale Veröffentlichungsnummer: WO 2019/170361

(56) Entgegenhaltungen:
- EP-A1- 3 150 190
- EP-A1- 3 260 113
- EP-A1- 3 378 537
- WO-A1-2011/101250
- WO-A1-2015/165715
- DE-A1-102015 219 006
- DATABASE GNPD [Online] MINTEL; 18. Juni 2014 (2014-06-18), anonymous: "Silky Milk SPF 15 - Body", XP055578287, gefunden im www.gnpd.com Database accession no. 2497301
- DATABASE GNPD [Online] MINTEL; 16. August 2017 (2017-08-16), anonymous: "Sun Stick SPF 50", XP55577885, gefunden im www.gnpd.com Database accession no. 5025319
- DATABASE GNPD [Online] MINTEL; 17. Oktober 2016 (2016-10-17), anonymous: "AR Tinted Cream SPF 50+", XP55577887, gefunden im www.gnpd.com Database accession no. 4347195
- DATABASE GNPD [Online] MINTEL; 15. Juni 2016 (2016-06-15), anonymous: "Protector Day Cream for Face and Decollete", XP55577881, gefunden im www.gnpd.com Database accession no. 4063431
- DATABASE GNPD [Online] MINTEL; 19. Januar 2018 (2018-01-19), anonymous: "Day Cream SPF 20", XP55577883, gefunden im www.gnpd.com Database accession no. 5391231

## Beschreibung

Die vorliegende Erfindung betrifft eine kosmetische Zubereitung enthaltend hydriertes Pflanzenöl, insbesondere hydriertes Rapsöl, und 2,4-Bis-{[4-(2-ethyl-hexyloxy)-2-hydroxy]-phenyl}-6-(4-methoxyphenyl)-1,3,5-triazin (INCI Bis-Ethylhexyloxyphenol Methoxyphenyl Triazine), sowie Verfahren und Verwendungen von hydriertem Pflanzenöl, insbesondere hydriertem Rapsöl, beim Schutz von Textilien vor dauerhaften Verfärbungen durch UV-Filter (insbesondere Bis-Ethylhexyloxyphenol Methoxyphenyl Triazine) enthaltende kosmetische Zubereitungen.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurde daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 2,4-Bis-{[4-(2-ethyl-hexyloxy)-2-hydroxy]-phenyl}-6-(4-methoxyphenyl)-1 ,3,5-triazin (INCI Bis-Ethylhexyloxyphenol Methoxyphenyl Triazine) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) enthaltend nicht-wasserlösliche Breitbandfilter wie Bis-Ethylhexyloxyphenol Methoxyphenyl Triazine zu entwickeln, welche sich leichter aus den mit der Zubereitung kontaminierten Textilen herauswaschen lassen.

Überraschend gelöst wird diese Aufgabe durch eine kosmetische Zubereitung enthaltend 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und hydriertes Pflanzenöl (INCI: hydrogenated Vegetable Oil), wobei die Zubereitung als hydriertes Pflanzenöl (INCI hydrogenated Vegetable Oil) hydriertes Rapsöl (INCI: hydrogenated Rapeseed Oil) enthält und die Zubereitung frei ist von Propyl- und Butylparaben, 3-lod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

Überraschend gelöst wird diese Aufgabe außerdem durch die Verwendung von hydriertem Pflanzenöl (INCI hydrogenated Vegetable Oil) in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, dadurch gekennzeichnet, dass die Zubereitung als hydriertes Pflanzenöl (INCI hydrogenated Vegetable Oil) hydriertes Rapsöl (INCI: hydrogenated Rapeseed Oil) enthält.

Zwar kennt der Stand der Technik die in der GNPD Datenbank "Mintel" offenbarten Produkte mit den Eintragungsnummern (Record ID) 2497301, 50025319, 4347195, 4063431 und 5391231 sowie die Veröffentlichungen WO 2015/165715 A1, EP 3260113 A1, WO 2011/101250 A1, DE 10 2015 219 006 A1 und EP 3150190 A1, doch konnten diese Offenbarungen nicht den Weg zur vorliegenden Erfindung weisen.

Die Verwendung betrifft erfindungsgemäß bevorzugt kosmetische Zubereitungen enthaltend kosmetische Zubereitung enthaltend 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin).

Im Rahmen der vorliegenden Offenbarung beziehen sich die Formulierungen "erfindungsgemäß", "erfindungsgemäße Zubereitung" etc. immer auf die erfindungsgemäßen Zubereitungen und Verwendungen, d.h. auch auf Zubereitungen, in denen die erfindungsgemäßen Verwendungen verwirklicht werden.

Vorteilhafte Ausführungsformen der vorliegenden Erfindung sind dadurch gekennzeichnet, dass die Zubereitung 4-(tert.-Butyl)-4'-methoxydibenzoylmethan enthält.

Erfindungsgemäß besonders bevorzugt ist es, wenn die Zubereitung als hydriertes Pflanzenöl (INCI hydrogenated Vegetable Oil) ausschließlich hydriertes Rapsöl (INCI: hydrogenated Rapeseed Oil) enthält.

Es ist erfindungsgemäß von Vorteil, wenn die Zubereitung hydriertes Rapsöl (INCI: hydrogenated Rapeseed Oil) in einer Konzentration von 0,1 bis 10 Gewichts-%, bezogen auf das Gesamtgewicht der Zubereitung, enthält. Dabei ist ein Konzentrationsbereich von 0,1 bis 7 Gewichts-%, bezogen auf das Gesamtgewicht der Zubereitung, erfindungsgemäß bevorzugt.

Ferner ist es erfindungsgemäß vorteilhaft, wenn die Zubereitung 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin) in einer Konzentration von 0,1 bis 10 Gewichts-%, bezogen auf das Gesamtgewicht der Zubereitung enthält. Dabei ist ein Konzentrationsbereich von 0,1 bis 7 Gewichts-%, bezogen auf das Gesamtgewicht der Zubereitung, erfindungsgemäß bevorzugt.

Vorteilhaft im Sinne der vorliegenden Erfindung ist es, wenn das Gewichtsverhältnis von 2,4-Bis-{[4-(2-ethyl-hexyloxy)-2-hydroxy]-phenyl}-6-(4-methoxyphenyl)-1,3,5-triazin zum hydrierten Rapsöl in der Zubereitung im Bereich von 0,01:1 bis 1 :0,01 gewählt wird.

Es ist für die erfindungsgemäße Verwendung erfindungsgemäß vorteilhaft, wenn die Zubereitung frei ist von Propyl- und Butylparaben, 3-lod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate). Erfindungsgemäß bevorzugt wird darüber hinaus auch auf Methyl- und Ethylparaben sowie auf 2,6-Dihydroxy-4-methylbenzaldehyd (Atranol) und 3-Chlor-2,6-dihydroxy-4-methylbenzaldehyd (Chloratranol) verzichtet. Nicht zuletzt ist es erfindungsgemäß von Vorteil, wenn die Zubereitung frei ist von Octocrylen.

Erfindungsgemäß von Vorteil ist es, wenn die Zubereitung 4-(tert.-Butyl)-4'-methoxydiben-zoylmethan in einer Konzentration von 0,1 bis 5 Gewichts-%, bezogen auf das Gesamtgewicht der Zubereitung enthält.

Vorteilhafte Ausführungsformen der vorliegenden Erfindung sind dadurch gekennzeichnet, dass die Zubereitung Ethanol in einer Konzentration von 0,1 bis 75 Gewichts-%, bezogen auf das Gesamtgewicht der Zubereitung enthält.

Die erfindungsgemäße Zubereitung kann in zwei unterschiedlichen Formen vorliegen: Als Emulsion und als ethanolische Lösung.

Liegt die Zubereitung als Emulsion vor, so ist es erfindungsgemäß von Vorteil, wenn diese Emulsion eine Öl-in-Wasser (O/W) Emulsion darstellt. In einem solchen Falle ist es erfindungsgemäß von Vorteil, wenn die Emulgatoren gewählt werden aus der Gruppe der Verbindungen Glycerylstearatcitrat, Glycerylstearat (selbstemulgierend), Stearinsäure, Stearatsalze, Polyglyceryl-3 methylglycose-distearat, Natriumcetearylsulfat, Kaliumcetylphosphat, Polyglyceryl-10 Stearate, Natriumstearoyl-glutamat.

Dabei sind die erfindungsgemäß vorteilhaften Ausführungsformen dadurch gekennzeichnet, dass der oder die Emulgatoren in einer Gesamtkonzentration von 0,1 bis 5 Gewichts-%, bezogen auf das Gesamtgewicht der Zubereitung eingesetzt werden.

Liegt die erfindungsgemäße Zubereitung hingegen als ethanolische Lösung vor, beträgt der Ethanolgehalt vorteilhaft von 20 bis 75 Gewichts-%, bezogen auf das Gesamtgewicht der Zubereitung.

Es ist erfindungsgemäß von Vorteil, wenn die Zubereitung Phenoxyethanol, Ethylhexylglycerin und/oder 4-Hydroxyacetophenon enthält. Dies gilt insbesondere für die erfindungsgemäßen Emulsionen.

Liegt die erfindungsgemäße Zubereitung in Form einer Emulsion vor, so kann die Wasserphase dieser Emulsion die hierfür üblichen Bestandteile enthalten. Darüber hinaus ist es erfindungsgemäß von Vorteil, wenn die Emulsion Propylenglykol, Butylenglycol, Glycerin, Elektrolyte, Selbstbräuner und/oder Verdickungsmittel enthält, beispielsweise Siliciumdioxide, Aluminiumsilikate oder Verbindungen aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist erfindungsgemäß bevorzugt, wenn die erfindungsgemäße Zubereitung Silica Dimethyl Silylate enthält.

Für alle Zubereitungsformen der vorliegenden Erfindung ist es von Vorteil, wenn die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Vorteilhaft ist es auch, wenn die erfindungsgemäße Emulsion Stärkederivate wie Tapiocastärke, Distärkephosphat und/oder Aerosile^{®} enthält.

Ferner kann die erfindungsgemäße Zubereitung vorteilhaft Filmbildner enthalten. Vorteilhafte fettlösliche Filmbildner sind z. B., die Filmbildner aus der Gruppe der Polymere auf Basis von Polyvinylpyrrolidon (PVP)

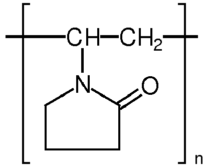

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Neben 2,4-Bis-{[4-(2-ethyl-hexyloxy)-2-hydroxy]-phenyl}-6-(4-methoxyphenyl)-1,3,5-triazin und gegebenenfalls 4-(tert.-Butyl)-4'-methoxydibenzoylmethan kann die erfindungsgemäße Zubereitung noch weitere für kosmetische Zubereitungen zugelassene UV-Filter enthalten. Dabei ist es erfindungsgemäß bevorzugt, wenn die Zubereitung einen oder mehrere UV-Filter aus der Gruppe der Verbindungen 2-(4'-Diethylamino-2'-hydoxybenzoyl)-benzoesäurehexylester, Homomenthylsalicylat, Ethylhexylsalicylat, Dioctylbutylamidotriazon (INCI: Diethylhexyl-Butamidotriazone), 2,4,6-Tris-[anilino-(p-carbo-2'-ethyl-1'-hexyloxy)]-1,3,5-triazin (INCI: Ethylhexyl Triazone), 2,2'-Methylen-bis-(6-(2H-benzotriazol-2-yl)-4-(1,1,3,3-tetramethylbutyl)-phenol), 2,4,6-Tribiphenyl-4-yl-1,3,5-triazin, Titandioxid, Phenylbenzimidazol-5-sulfonsäure und/oder deren Salze, [(3*Z*)-3-[[4-[(*Z*)-[7,7-Dimethyl-2-oxo-1-(sulfomethyl)-3-bicyclo[2.2.1]heptanyliden] methyl]phenyl]methyliden]-7,7-dimethyl-2-oxo-1-bicyclo[2.2.1]heptanyl]methan sulfonsäure (INCI: Terephthalylidene dicamphor sulfonic acid) und/oder dessen Salze enthält.

Es ist erfindungsgemäß von Vorteil, wenn die erfindungsgemäße Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Erfindungsgemäß vorteilhafte Ausführungsformen der vorliegenden Erfindung sind auch dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Es ist erfindungsgemäß von Vorteil, wenn die erfindungsgemäße Zubereitung einen oder mehrere der Parfümstoffe gewählt aus der Liste der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C, Butylphenylmethyl-propionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Ethylenbrassylat, Evernia Furfuracea Extract, Evernia Prunastri Extract, Farnesol, Guajakholzöl, Geraniol, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin, enthält.

Vorteilhafte Ausführungsformen der vorliegenden Erfindung sind dadurch gekennzeichnet, dass die Zubereitung Gumen und/oder Cellulose-Derivate enthält. Erfindungsgemäß bevorzugte Gumen sind Welan Gum, Sclerotium Gum und insbesondere Xanthan Gum.

Erfindungsgemäß bevorzugte Cellulose-Derivate sind mikrokristalline Cellulose und insbesondere Carboxymethylcellulose (Cellulose Gum).

Es ist vorteilhaft im Sinne der vorliegenden Erfindung, wenn die Zubereitung ein oder mehrere der Säure- bzw. Salzverbindungen aus der Gruppe 1-Hydroxyethan-(1,1-diphosphonsäure)/ HEDP, Aminotrimethylenphosphonsäure/ ATMP, Diethylentriaminpenta(methylenphosphonsäure)/ DTPMP, Ethylendiamintetra(methylenphosphonsäure/ EDTMP, Phosphonobutan-tricarbonsäure/ PBTC, Iminodisuccinat, Natriumpolyphosphat, Tetranatriumpyrophosphat, Hydroxamsäure, Polygalacturonsäure, Bernsteinsäure, Ameisensäure, Äpfelsäure, Ethylendiamintetraessigsäure (EDTA) und/oder deren Alkalisalze und/ oder deren Amin-N-Oxide enthält.

Erfindungsgemäß bevorzugt ist es dabei, wenn die Zubereitung die Natriumsalze der Ethylendiamintetraessigsäure und/oder der Iminodibernsteinsäure enthält. Besonders bevorzugt ist die Kombination aus den Natriumsalzen der Ethylendiamintetraessigsäure und der Iminodibernsteinsäure.

### Vergleichsversuch

Mit Hilfe des folgenden Vergleichsversuches konnte der erfinderische Effekt beispielhaft belegt werden: Es wurden die folgenden Rezepturen hergestellt und die Auswaschbarkeit aus Textilien bestimmt.

| **INCI** | **Referenz** | **+ Hydrogenated Rapeseed Oil** |
|---|---|---|
| Tocopheryl Acetate | 0,06 | 0,06 |
| Hydroxyacetophenone | 0,4 | 0,4 |
| Panthenol + Aqua | 1,4 | 1,4 |
| C12-15 Alkyl Benzoate | 2,5 | 2,0 |
| Butylene Glycol Dicaprylate/Dicaprate | 2,5 | 2,0 |
| Hydrogenated Rapeseed Oil | 0 | 1,0 |
| Sodium Stearoyl Glutamate | 0,25 | 0,25 |
| Silica Dimethyl Silylate | 1,0 | 1,0 |
| VP/Hexadecene Copolymer | 0,5 | 0,5 |
| Parfum | 0,4 | 0,4 |
| Glycerin + Aqua | 7,5 | 7,5 |
| Aqua + Sodium Hydroxide | 0,014 | 0,014 |
| Phenoxyethanol | 0,5 | 0,5 |
| Behenyl Alcohol | 0,9 | 0,9 |
| Stearyl Alcohol | 0,9 | 0,9 |
| Cellulose Gum | 0,5 | 0,5 |
| Xanthan Gum | 0,4 | 0,4 |
| Aqua | 48,776 | 48,776 |
| Alcohol Denat. + Aqua | 5,0 | 5,0 |
| Aqua + Trisodium EDTA | 1,0 | 1,0 |
| Tetrasodium Iminodisuccinate | 0,75 | 0,75 |
| Diethylamino Hydroxybenzoyl Hexyl Benzoate | 0,5 | 0,5 |
| Homosalate | 9,5 | 9,5 |
| Ethylhexyl Salicylate | 2,0 | 2,0 |
| Bis-Ethylhexyloxyphenol | | |
| Methoxyphenyl Triazine | 3,5 | 3,5 |
| Ethylhexyl Triazone | 2,0 | 2,0 |
| Butyl Methoxydibenzoylmethane | 4,75 | 4,75 |
| Titanium Dioxide (nano) + Silica + Dimethicone | 2,0 | 2,0 |
| Phenylbenzimidazole Sulfonic Acid | 0,5 | 0,5 |

### Messung der Auswaschbarkeit

Es wurden die beiden Sonnenschutzemulsionen hinsichtlich der Bildung von gelben Flecke über einen in vitro Auftragungs-/ Waschzyklus untersucht. Es wurden dabei weiße vorgewaschene Baumwollmonitore (100% Baumwolle) verwendet. Dazu wurden je 3 mg/cm2 der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät DATACOLOR 800 (Datacolor International).
Messgeometrie: d/8°, SCE (Glanzkomponente ausgeschlossen)
Lichtart/Beobachter: D65/10°(entsprechend mittleres Tageslicht)
UV-Filterung: an D65 angepasst, Ganz/Griesser Methode
Messöffnung: LAV (30 mm Durchmesser)
Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen in einer Waschmaschine) (60°C, 2h, Ariel Compact Pulverwaschmittel,saubere Beiladung).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät DATACOLOR 800 (Datacolor International).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

### Ergebnis

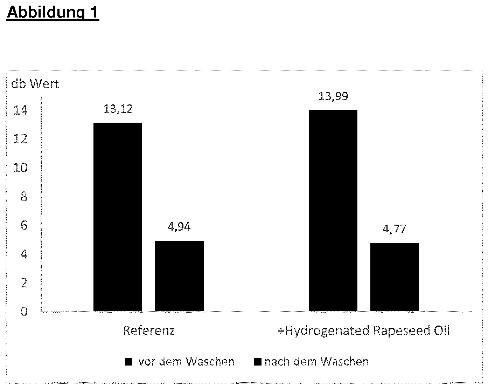

Während der db-Wert der Referenz-Rezeptur vor dem Waschen bei 13,12 liegt und nach dem Waschen auf 4,94 absinkt, geht dieser Wert bei der erfindungsgemäßen Zubereitung mit Hydrogenated Rapeseed Oil von 13,99 (vor dem Waschen) auf 4,77 (nach dem Waschen) zurück (siehe Abbildung 1).

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.
1) Sun Lotion SPF20

| **INCI** | **m(%)** |
|---|---|
| Tocopheryl Acetate | 0,1 |
| Hydroxyacetophenone | 0,4 |
| Panthenol | 1,5 |
| Ethylhexylglycerin | 0,3 |
| C12-15 Alkyl Benzoate | 5,0 |
| Isopropyl Palmitate | 6,0 |
| Butylene Glycol Dicaprylate/Dicaprate | 2,0 |
| Dimethicone | 0,9 |
| C18-38 Alkyl Hydroxystearoyl Stearate | 1,0 |
| Hydrogenated Rapeseed Oil | 0,75 |
| Hydrogenated Vegetable Oil | 0,3 |
| Polyglyceryl-10 Stearate | 0,4 |
| Silica Dimethyl Silylate | 1,0 |
| Triacontanyl PVP | 0,25 |
| VP/Hexadecene Copolymer | 0,25 |
| Parfum | 0,5 |
| Glycerin | 7,0 |
| Sodium Hydroxide | q.s |
| Behenyl Alcohol | 1,0 |
| Stearyl Alcohol | 1,0 |
| Acrylates/C1 0-30 Alkyl Acrylate Crosspolymer | 0,1 |
| Xanthan Gum | 0,4 |
| Aqua | add to 100 |
| Alcohol Denat. | 4,0 |
| Trisodium EDTA | 0,25 |
| Diethylamino Hydroxybenzoyl Hexyl Benzoate | 0,4 |
| Ethylhexyl Salicylate | 3,0 |
| Bis-Ethylhexyloxyphenol Methoxyphenyl Triazine | 0,33 |
| Ethylhexyl Triazone | 2,0 |
| Butyl Methoxydibenzoylmethane | 3,2 |
| Phenylbenzimidazole Sulfonic Acid | 0,8 |

2) Sun Lotion SPF 30

| **INCI** | **m(%)** |
|---|---|
| Tocopheryl Acetate | 0,06 |
| Hydroxyacetophenone | 0,4 |
| Panthenol | 1,1 |
| C12-15 Alkyl Benzoate | 5,0 |
| Isopropyl Palmitate | 6,0 |
| Butylene Glycol Dicaprylate/Dicaprate | 2,0 |
| Hydrogenated Rapeseed Oil | 1,0 |
| Sodium Stearoyl Glutamate | 0,25 |
| Silica Dimethyl Silylate | 1,0 |
| VP/Hexadecene Copolymer | 0,5 |
| Parfum | 0,4 |
| Glycerin | 7,5 |
| Sodium Hydroxide | q.s |
| Phenoxyethanol | 0,5 |
| Behenyl Alcohol | 1,0 |
| Stearyl Alcohol | 1,0 |
| Cellulose Gum | 0,35 |
| Xanthan Gum | 0,4 |
| Aqua | add to 100 |
| Alcohol Denat. | 5,0 |
| Trisodium EDTA | 0,2 |
| Tetrasodium Iminodisuccinate | 0,5 |
| Diethylamino Hydroxybenzoyl Hexyl Benzoate | 0,5 |
| Ethylhexyl Salicylate | 2,5 |
| Bis-Ethylhexyloxyphenol Methoxyphenyl Triazine | 2,2 |
| Ethylhexyl Triazone | 0,75 |
| Butyl Methoxydibenzoylmethane | 2,4 |
| Titanium Dioxide (nano) + Silica + Dimethicone | 3,0 |
| Phenylbenzimidazole Sulfonic Acid | 0,5 |

3) Sun Spray SPF 50+

| **INCI** | **m(%)** |
|---|---|
| Tocopheryl Acetate | 0,06 |
| Panthenol | 1,0 |
| Ethylhexylglycerin | 0,3 |
| Hydrogenated Rapeseed Oil | 0,5 |
| Dibutyl Adipate | 3,0 |
| Butylene Glycol Dicaprylate/Dicaprate | 0,5 |
| Copernicia Cerifera Cera | 1,5 |
| Sucrose Polystearate + Hydrogenated Polyisobutene | 1,0 |
| Polyglyceryl-4 Diisostearate/Polyhydroxystearate/Sebacate | 0,5 |
| Sodium Stearoyl Glutamate | 0,4 |
| VP/Hexadecene Copolymer | 1,0 |
| Parfum | 0,5 |
| Glycerin | 5,0 |
| Sodium Hydroxide | q.s |
| Phenoxyethanol | 0,5 |
| Cellulose Gum | 0,5 |
| Acrylates/C1 0-30 Alkyl Acrylate Crosspolymer | 0,1 |
| Xanthan Gum | 0,15 |
| Microcrystalline Cellulose + Cellulose Gum | 1,0 |
| Aqua | Add to 100 |
| Alcohol Denat. | 5,0 |
| Tetrasodium Iminodisuccinate | 0,75 |
| Trisodium EDTA | 0,2 |
| Homosalate | 8,0 |
| Ethylhexyl Salicylate | 8,5 |
| Bis-Ethylhexyloxyphenol Methoxyphenyl Triazine | 3,2 |
| Ethylhexyl Triazone | 2,8 |
| Butyl Methoxydibenzoylmethane | 5,00 |
| Phenvlbenzimidazole Sulfonic Acid | 0,5 |

## Patentansprüche

1. Kosmetische Zubereitung enthaltend 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) und hydriertes Pflanzenöl (INCI: hydrogenated Vegetable Oil), wobei die Zubereitung als hydriertes Pflanzenöl (INCI hydrogenated Vegetable Oil) hydriertes Rapsöl (INCI: hydrogenated Rapeseed Oil) enthält und die Zubereitung frei ist von Propyl- und Butylparaben, 3-lod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

2. Verwendung von hydriertem Pflanzenöl (INCI hydrogenated Vegetable Oil) in kosmetischen Zubereitungen enthaltend organische, öllösliche UV-A Filter und/oder Breitbandfilter zur Erhöhung der Auswaschbarkeit dieser UV-Filter aus mit der Zubereitung kontaminierten Textilien, **dadurch gekennzeichnet, dass** die Zubereitung als hydriertes Pflanzenöl (INCI hydrogenated Vegetable Oil) hydriertes Rapsöl (INCI: hydrogenated Rapeseed Oil) enthält.

3. Verwendung nach Anspruch 2, **dadurch gekennzeichnet, dass** die kosmetische Zubereitung 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthält.

4. Kosmetische Zubereitung oder Verwendung nach einem der vorhergehenden Ansprüche, **dadurch gekennzeichnet, dass** die Zubereitung 4-(tert.-Butyl)-4'-methoxy-dibenzoylmethan enthält.

5. Kosmetische Zubereitung oder Verwendung nach einem der vorhergehenden Ansprüche, **dadurch gekennzeichnet, dass** die Zubereitung als hydriertes Pflanzenöl (INCI hydrogenated Vegetable Oil) ausschließlich hydriertes Rapsöl (INCI: hydrogenated Rapeseed Oil) enthält.

6. Kosmetische Zubereitung oder Verwendung nach einem der vorhergehenden Ansprüche, **dadurch gekennzeichnet, dass** die Zubereitung hydriertes Rapsöl (INCI: hydrogenated Rapeseed Oil) in einer Konzentration von 0,1 bis 10 Gewichts-%, bezogen auf das Gesamtgewicht der Zubereitung, enthält.

7. Kosmetische Zubereitung oder Verwendung nach einem der vorhergehenden Ansprüche, **dadurch gekennzeichnet, dass** die Zubereitung 2,4-Bis-{[4-(2-ethyl-hexyl-oxy)-2-hydroxy]-phenyl}-6-(4-methoxyphenyl)-1,3,5-triazin (INCI: Bis-Ethylhexyloxyphenol Methoxyphenyl Triazin) in einer Konzentration von 0,1 bis 10 Gewichts-%, bezogen auf das Gesamtgewicht der Zubereitung enthält.

8. Kosmetische Zubereitung oder Verwendung nach einem der vorhergehenden Ansprüche, **dadurch gekennzeichnet, dass** das Gewichtsverhältnis von 2,4-Bis-{[4-(2-ethyl-hexyloxy)-2-hydroxy]-phenyl}-6-(4-methoxyphenyl)-1,3,5-triazin zum hydrierten Rapsöl in der Zubereitung im Bereich von 0,01:1 bis 1:0,01 gewählt wird.

9. Kosmetische Zubereitung oder Verwendung nach einem der vorhergehenden Ansprüche, **dadurch gekennzeichnet, dass** die Zubereitung 4-(tert.-Butyl)-4'-methoxy-dibenzoylmethan in einer Konzentration von 0,1 bis 5 Gewichts-%, bezogen auf das Gesamtgewicht der Zubereitung enthält.

10. Verwendung nach einem der vorhergehenden Ansprüche, **dadurch gekennzeichnet, dass** die Zubereitung frei ist von Propyl- und Butylparaben, 3-lod-2-propinylbutylcarbamat, Hydroxyisohexyl 3-Cyclohexene Carboxalhdehyde (Lyral), 3-(4-Methylbenzyliden)-campher, 2-Hydroxy-4-methoxybenzophenon (Oxybenzon) und 4-Methoxyzimtsäure-2-ethylhexylester (Ethylhexyl Methoxycinnamate).

11. Kosmetische Zubereitung oder Verwendung nach einem der vorhergehenden Ansprüche, **dadurch gekennzeichnet, dass** die Zubereitung Ethanol in einer Konzentration von 0,1 bis 75 Gewichts-%, bezogen auf das Gesamtgewicht der Zubereitung enthält.

12. Kosmetische Zubereitung oder Verwendung nach einem der vorhergehenden Ansprüche, **dadurch gekennzeichnet, dass** die Zubereitung Phenoxyethanol, Ethylhexylglycerin und/oder 4-Hydroxyacetophenon enthält.

13. Kosmetische Zubereitung oder Verwendung nach einem der vorhergehenden Ansprüche, **dadurch gekennzeichnet, dass** die Zubereitung einen oder mehrere UV-Filter aus der Gruppe der Verbindungen 2-(4'-Diethylamino-2'-hydoxybenzoyl)-benzoesäurehexylester, Homomenthylsalicylat, Ethylhexylsalicylat, Dioctylbutylamidotriazon (INCI: Diethylhexyl-Butamidotriazone), 2,4,6-Tris-[anilino-(p-carbo-2'-ethyl-1'-hexyloxy)]-1,3,5-triazin (INCI: Ethylhexyl Triazone), 2,2'-Methylen-bis-(6-(2H-benzotriazol-2-yl)-4-(1,1,3,3-tetramethylbutyl)-phenol), 2,4,6-Tribiphenyl-4-yl-1,3,5-triazin, Titandioxid, Phenylbenzimidazol-5-sulfonsäure und/oder deren Salze, [(3Z)-3-[[4-[(Z)-[7,7-Dimethyl-2-oxo-1-(sulfomethyl)-3-bicyclo[2.2.1]heptanyliden] methyl]phenyl]methyliden]-7,7-dimethyl-2-oxo-1-bicyclo[2.2.1]heptanyl]methan sulfonsäure (INCI: Terephthalylidene dicamphor sulfonic acid) und/oder dessen Salze enthält.

14. Kosmetische Zubereitung oder Verwendung nach einem der vorhergehenden Ansprüche, **dadurch gekennzeichnet, dass** die Zubereitung Gumen und/oder Cellulose-Derivate enthält.

15. Kosmetische Zubereitung oder Verwendung nach einem der vorhergehenden Ansprüche, **dadurch gekennzeichnet, dass** die Zubereitung die Natriumsalze der Ethylendiamintetraessigsäure und/oder der Iminodibernsteinsäure enthält.

## Claims

1. Cosmetic preparation comprising 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and hydrogenated vegetable oil (INCI: Hydrogenated Vegetable Oil), the preparation comprising hydrogenated rapeseed oil (INCI: Hydrogenated Rapeseed Oil) as hydrogenated vegetable oil (INCI: Hydrogenated Vegetable Oil) and the preparation being free of propyl and butyl paraben, 3-iodo-2-propynyl butylcarbamate, Hydroxyisohexyl 3-Cyclohexene Carboxaldehyde (Lyral), 3-(4-methylbenzylidene)camphor, 2-hydroxy-4-methoxybenzophenone (Oxybenzone) and 2-ethylhexyl 4-methoxycinnamate (Ethylhexyl Methoxycinnamate).

2. Use of hydrogenated vegetable oil (INCI: Hydrogenated Vegetable Oil) in cosmetic preparations comprising organic, oil-soluble UV-A filters and/or broad-spectrum filters in order to increase the ability to wash these UV filters out of textiles contaminated with the preparation, **characterized in that** the preparation comprises hydrogenated rapeseed oil (INCI: Hydrogenated Rapeseed Oil) as hydrogenated vegetable oil (INCI: Hydrogenated Vegetable Oil).

3. Use according to Claim 2, **characterized in that** the cosmetic preparation comprises 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

4. Cosmetic preparation or use according to any of the preceding claims, **characterized in that** the preparation comprises 4-(tert-butyl)-4'-methoxydibenzoylmethane.

5. Cosmetic preparation or use according to any of the preceding claims, **characterized in that** the preparation comprises exclusively hydrogenated rapeseed oil (INCI: Hydrogenated Rapeseed Oil) as hydrogenated vegetable oil (INCI: Hydrogenated Vegetable Oil).

6. Cosmetic preparation or use according to any of the preceding claims, **characterized in that** the preparation comprises hydrogenated rapeseed oil (INCI: Hydrogenated Rapeseed Oil) at a concentration of 0.1% to 10% by weight, based on the total weight of the preparation.

7. Cosmetic preparation or use according to any of the preceding claims, **characterized in that** the preparation comprises 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) at a concentration of 0.1% to 10% by weight, based on the total weight of the preparation.

8. Cosmetic preparation or use according to any of the preceding claims, **characterized in that** the weight ratio of 2,4-bis{[4-(2-ethylhexyloxy)-2-hydroxy]phenyl}-6-(4-methoxyphenyl)-1,3,5-triazine to hydrogenated rapeseed oil in the preparation is selected in the range from 0.01:1 to 1:0.01.

9. Cosmetic preparation or use according to any of the preceding claims, **characterized in that** the preparation comprises 4-(tert-butyl)-4'-methoxydibenzoylmethane at a concentration of 0.1% to 5% by weight, based on the total weight of the preparation.

10. Use according to any of the preceding claims, **characterized in that** the preparation is free of propyl and butyl paraben, 3-iodo-2-propynyl butylcarbamate, Hydroxyisohexyl 3-Cyclohexene Carboxaldehyde (Lyral), 3-(4-methylbenzylidene)camphor, 2-hydroxy-4-methoxybenzophenone (Oxybenzone) and 2-ethylhexyl 4 methoxycinnamate (Ethylhexyl Methoxycinnamate).

11. Cosmetic preparation or use according to any of the preceding claims, **characterized in that** the preparation comprises ethanol at a concentration of 0.1% to 75% by weight, based on the total weight of the preparation.

12. Cosmetic preparation or use according to any of the preceding claims, **characterized in that** the preparation comprises phenoxyethanol, ethylhexylglycerin and/or 4-hydroxyacetophenone.

13. Cosmetic preparation or use according to any of the preceding claims, **characterized in that** the preparation comprises one or more UV filters from the following group of compounds: hexyl 2-(4'-diethylamino-2'-hydroxybenzoyl)benzoate, homomenthyl salicylate, ethylhexyl salicylate, dioctylbutylamidotriazone (INCI: Diethylhexyl Butamido Triazone), 2,4,6-tris[anilino(p-carbo-2'-ethyl-1'-hexyloxy)]-1,3,5-triazine (INCI: Ethylhexyl Triazone), 2,2'-methylenebis(6-(2H-benzotriazol-2-yl)-4-(1,1,3,3-tetramethylbutyl)phenol), 2,4,6-tribiphenyl-4-yl-1,3,5-triazine, titanium dioxide, phenylbenzimidazole-5-sulfonic acid and/or salts thereof, [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI: Terephthalylidene Dicamphor Sulfonic Acid) and/or salts thereof.

14. Cosmetic preparation or use according to any of the preceding claims, **characterized in that** the preparation comprises gums and/or cellulose derivatives.

15. Cosmetic preparation or use according to any of the preceding claims, **characterized in that** the preparation comprises the sodium salts of ethylenediaminetetraacetic acid and/or of iminodisuccinic acid.

## Revendications

1. Préparation cosmétique contenant de la 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) et de l'huile végétale hydrogénée (INCI : hydrogenated Vegetable Oil), la préparation contenant, en tant qu'huile végétale hydrogénée (INCl hydrogenated Vegetable Oil), de l'huile de canola hydrogénée (INCI : hydrogenated Rapeseed Oil) et la préparation étant exempte de propylparabène et de butylparabène, de 3-iodo-2-propynylbutylcarbamate, d'hydroxyisohexyl-3-cyclohexènecarboxaldéhyde (Lyral), de 3-(4-méthylbenzylidène)-camphre, de 2-hydroxy-4-méthoxybenzophénone (Oxybenzone) et d'ester 2-éthylhexylique de l'acide 4-méthoxycinnamique (Ethylhexyl Methoxycinnamate).

2. Utilisation d'huile végétale hydrogénée (INCl hydrogenated Vegetable Oil) dans des préparations cosmétiques contenant des filtres UV-A et/ou des filtres large bande organiques, solubles dans l'eau pour augmenter la rinçabilité de ces filtres UV à partir de textiles contaminés par la préparation, **caractérisée en ce que** la préparation contient, en tant qu'huile végétale hydrogénée (INCl hydrogenated Vegetable Oil), de l'huile de canola hydrogénée (INCI : hydrogenated Rapeseed Oil).

3. Utilisation selon la revendication 2, **caractérisée en ce que** la préparation cosmétique contient de la 2,4-bis-{[4-(2-éthylhexyloxy)-2-hydroxy]phényl}-6-(4-méthoxyphényl)-1,3,5-triazine (INCI : Bis-Ethylhexyloxyphenol Methoxyphenyl Triazin).

4. Préparation cosmétique ou utilisation selon l'une quelconque des revendications précédentes, **caractérisée en ce que** la préparation contient du 4-(tert-butyl)-4'-méthoxydibenzoylméthane.

5. Préparation cosmétique ou utilisation selon l'une quelconque des revendications précédentes, **caractérisée en ce que** la préparation contient, en tant qu'huile végétale hydrogénée (INCl hydrogenated Vegetable Oil), exclusivement de l'huile de canola hydrogénée (INCI : hydrogenated Rapeseed Oil).

6. Préparation cosmétique ou utilisation selon l'une quelconque des revendications précédentes, **caractérisée en ce que** la préparation contient de l'huile de canola hydrogénée (INCI : hydrogenated Rapeseed Oil) en une concentration de 0,1 à 10% en poids, par rapport au poids total de la préparation.

7. Préparation cosmétique ou utilisation selon l'une quelconque des revendications précédentes, **caractérisée en ce que** la préparation contient de la 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazin) en une concentration de 0,1 à 10% en poids, par rapport au poids total de la préparation.

8. Préparation cosmétique ou utilisation selon l'une quelconque des revendications précédentes, **caractérisée en ce que** le rapport pondéral de la 2,4-bis-{[4-(2-éthylhexyloxy)-2-hydroxy]phényl}-6-(4-méthoxyphényl)-1,3,5-triazine à l'huile de canola hydrogénée dans la préparation est choisi dans la plage de 0,01:1 à 1:0,01.

9. Préparation cosmétique ou utilisation selon l'une quelconque des revendications précédentes, **caractérisée en ce que** la préparation contient du 4-(tert-butyl)-4'-méthoxydibenzoylméthane en une concentration de 0,1 à 5% en poids, par rapport au poids total de la préparation.

10. Utilisation selon l'une quelconque des revendications précédentes, **caractérisée en ce que** la préparation est exempte de propylparabène et de butylparabène, de 3-iodo-2-propynylbutylcarbamate, d'hydroxyisohexyl-3-cyclohexènecarboxalhdéhyde (Lyral), de 3-(4-méthylbenzylidène)-camphre, de 2-hydroxy-4-méthoxybenzophénone (Oxybenzone) et d'ester 2-éthylhexylique de l'acide 4-méthoxycinnamique (Ethylhexyl Methoxycinnamate).

11. Préparation cosmétique ou utilisation selon l'une quelconque des revendications précédentes, **caractérisée en ce que** la préparation contient de l'éthanol en une concentration de 0,1 à 75% en poids par rapport au poids total de la composition.

12. Préparation cosmétique ou utilisation selon l'une quelconque des revendications précédentes, **caractérisée en ce que** la préparation contient du phénoxyéthanol, de l'éthylhexylglycérol et/ou de la 4-hydroxyacétophénone.

13. Préparation cosmétique ou utilisation selon l'une quelconque des revendications précédentes, **caractérisée en ce que** la préparation contient un ou plusieurs filtres UV du groupe des composés ester hexylique de l'acide 2-(4'-diéthylamino-2'-hydroxybenzoyl)-benzoïque, salicylate d'homomenthyle, salicylate d'éthylhexyle, dioctylbutylamidotriazone (INCI : Diethylhexylbutamidotriazone), 2,4,6-tris-[anilino-(p-carbo-2'-éthyl-1'-hexyloxy)]-1,3,5-triazine (INCI : Ethylhexyl Triazone), 2,2'-méthylène-bis-(6-(2H-benzotriazol-2-yl)-4-(1,1,3,3-tétraméthylbutyl)-phénol), 2,4,6-tribiphényl-4-yl-1,3,5-triazine, dioxyde de titane, acide phénylbenzimidazole-5-sulfonique et/ou ses sels, acide [(3Z)-3[[4-[(Z)[7,7-diméthyl-2-oxo-1-(sulfométhyl)-3-bicyclo[2,2,1]heptanylidène]méthyl]phényl]méthylidène]-7,7-diméthyl-2-oxo-1-bicyclo[2.2.1]heptanyl]méthanesulfonique (INCI : Terephtalylidene dicamphor sulfonic acid) et/ou ses sels.

14. Préparation cosmétique ou utilisation selon l'une quelconque des revendications précédentes, **caractérisée en ce que** la préparation contient des gommes et/ou des dérivés de cellulose.

15. Préparation cosmétique ou utilisation selon l'une quelconque des revendications précédentes, **caractérisée en ce que** la préparation contient les sels sodiques de l'acide éthylènediaminetétraacétique et/ou de l'acide iminodisuccinique.
